(19) Europäisches Patentamt
European Patent Office
Office européen des brevets

(11) Veröffentlichungsnummer: **0 177 444 B1**

(12) **EUROPÄISCHE PATENTSCHRIFT**

(45) Veröffentlichungstag der Patentschrift: **16.08.90**

(51) Int. Cl.⁵: **C 08 G 59/18**, C 08 G 59/50, C 08 G 18/58

(21) Anmeldenummer: **85810424.3**

(22) Anmeldetag: **18.09.85**

# (54) Addukte auf Toluoldiaminbasis als Härter für Epoxid- und Urethanharze.

(30) Priorität: **24.09.84 US 653417**

(43) Veröffentlichungstag der Anmeldung:
**09.04.86 Patentblatt 86/15**

(45) Bekanntmachung des Hinweises auf die Patenterteilung:
**16.08.90 Patentblatt 90/33**

(84) Benannte Vertragsstaaten:
**BE CH DE FR GB IT LI NL**

(56) Entgegenhaltungen:
**EP-A-0 044 816**
**FR-A-1 378 591**
**FR-A-2 164 887**
**FR-A-2 452 514**
**FR-A-2 487 371**
**GB-A-1 259 692**
**US-A-4 120 913**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Ham, Nancy M.**
**1917 Maple Shade**
**Williamston, MI 48895 (US)**

**EP 0 177 444 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Addukte von Aminen an Mono- und Diepoxide werden seit langem in der Industrie als Härter für Epoxidharze verwendet. Durch die Bildung solcher Addukte lassen sich Vorteile, wie beispielsweise niedrigere Flüchtigkeit, ein verringertes Reizpotential oder eine verminderte Tendenz zum Verfärben oder Ausschwitzen erzielen.

Derartige Addukte und die Vorteile, die sich damit erreichen lassen, werden im 'Handbook of Epoxy Resins' von Lee und Neville (McGraw Hill (1967)) diskutiert.

Es sei jedoch erwähnt, dass solche Addukte u.U. verglichen mit den entsprechenden freien Aminen keine erhöhte chemische Resistenz aufweisen.

Solche Addukte werden beispielsweise durch Umsetzung von Diethylentriamin mit Diglycidylethern von Bisphenol A unterschiedlichen Molekulargewichtes hergestellt. Diese Epoxidharze haben keine höhere Funktionalität als 2 und sie besitzen verschiedene vorteilhafte Eigenschaften. Wie bereits erwähnt, fehlt ihnen jedoch die wichtige Eigenschaft der chemischen Resistenz, insbesondere der Resistenz gegenüber Lösungsmitteln.

Ueblicherweise wird das Methylendianilinaddukt an Diglycicydylether von Bisphenol A als Härter für hitzeresistente Epoxidharze verwendet. Methylendianilin ist jedoch durch die Environmental Protection Agency als Karzinogen eingestuft.

Die US—PSS 3,655,624, 3,704,281 und 3,996,186 beschrieben Addukte von Triglycidylisocyanurat und Aminen. Diese Addukte sind Feststoffe und werden zur Verwendung in Pressmassen vorgeschlagen. Bezüglich der Lösungsmittelbeständigkeit weisen diese Produkte im allgemeinen nur mässige Eigenschaften auf und sind daher als Schutzüberzüge nur von begrenztem Wert.

In den US—PSS 3,538,184, 3,625,918 und 3,629,181 werden Aminaddukte vorgeschlagen, die auf Epoxidharzen vom Bisphenol A Typ oder auf alicyclischen Epoxidharzen basieren. Solche Addukte gestatten zwar eine verbesserte Handhabung und sie weisen verbesserte mechanische Eigenschaften auf, es bleibt jedoch festzustellen, dass die darin verwendeten Harze eine Funktionalität von nicht mehr als 2 aufweisen und dass die Addukte wiederum die wichtige Eigenschaft der Resistenze gegenüber chemischem Angriff vermissen lassen.

Weitere dieser Systeme werden in den US—PSS 3,996, 175 und 4,348,505 beschrieben.

Die obige Diskussion lässt erkennen, dass ein Bedarf an neuen flüssigen Addukten von Aminen und Di- und Polyepoxiden besteht.

Ferner besteht ein Bedarf an Addukten, die sich als Härter für Epoxidharze und für Urethanharze einsetzen lassen. Solche Härter sollten insbesondere die Nachteile der vorbekannten Härter nicht aufweisen, und sie sollten mit einer grossen Vielfalt von Epoxidharzen oder Urethanharzen kombiniert werden können.

Ueberraschenderweise wurde nun gefunden, dass die Eigenschaften gehärteter Epoxid- und Urethanharze verbessert werden können, wenn man als Härter Addukte von Diethyltoluoldiamin an Epoxide mit einer Funktionalität von mindestens zwei oder un Urethane verwendet.

Das vorliegende Addukt verkürzt die Härtungsdauer von Epoxidharzen beträchtlich, ohne die physikalischen Eigenschaften der gehärteten Produkte zu verschlechtern. Dies steht im Gegensatz zu den entsprechenden freien Aminhärtern, die wegen der begrenzten Reaktivität mit Epoxidharzen bei Raumtemperatur eine ausgesprochen lange Härtungsdauer benötigen, bis die Härtung zur B-Stufe erfolgt ist.

Von besagten Addukten können Härterformulierungen hergestellt werden, die zu einer Härtung bei Raumtemperatur führen, oder die zu einer B-Stufenhärtung bei Raumtemperatur führen und ein erneute Fliessfähigkeit bei erhöhten Temperaturen gestatten. Härterformulierungen, die diese Addukte enthalten, sind insbesondere nützlich bei der Herstellung wärmehärtbarer Laminate, wo der Einsatz eines wiederfliessfähigen Systems das Erreichen optimaler Eigenschaften gestattet.

Die wiederfliessfähigen Härter der vorliegenden Erfindung führen zu einem halbfesten B-Stufenprodukt bei Raumtemperatur und gehen bei erhöhten Temperaturen in einen flüssigen Zustand über.

Wenn solche wiederfliessfähigen Härter in einem Laminat eingesetzt werden, durchdringen sie die Fasern vollständig und Lufteinschlüsse werden auf diese Weise vermieden.

Laminate ohne Lufteinschlüsse können, verglichen mit solchen, die Lufteinschlüsse aufweisen, bei höheren Temperaturen eingesetzt werden und besitzen eine erhöht Stabilität.

Darüber hinaus werden diese Härteraddukte nicht als karzinogen angesehen, sie führen zu keinen Verfärbungen und sie sind bei Raumtemperatur flüssig.

Diethyltoluoldiamin der Formel I

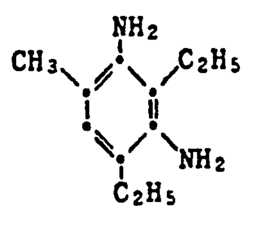

(I)

ist die dominierende Diaminkomponente. Es sei jedoch darauf hingewiesen, dass auch weitere Positionsisomere, beispielsweise das 2-Methyl-4,6-diethyl-1,3-diamino-benzol, in Kombination mit dem Isomer der Formel I vorliegen können, um als Diaminkomponente für die Zwecke der vorliegenden Erfindung zu dienen.

Kommerziell verfügbare Diamine enthalten in der Regel 75 Gew.% des 2,4-Diethylisomeren und etwa 20 Gew.% des 4,6-Diethylisomeren. Der Rest besteht in der Regel aus weiteren verwandten Verbindungen. So ist beispielsweise ein kommerzielles Diethyltoluolidiamingemisch erhältlich, das zu 75,5 Gew.% aus 3,5-Diethyltoluol-2,4-diamin, zu 21,0 Gew.% aus 3,5-Diethyltoluol-2,6-diamin, zu 3,0 Gew.% aus dialkyliertem m-Phenylendiamin, zu 0,4 Gew.% aus weiteren trialkylierten m-Phenylendiaminen und zu 0,1 Gew.% aus weiteren Verbindungen besteht.

Die multifuntkionelle Epoxidkomponente kann aus dem breiten Bereich der aliphatischen und aromatischen Epoxidverbindungen mit einer Funktionalität von wenigstens zwei ausgewählt werden.

Zu den typischen Materialien dieser Gruppe zählen beispeilsweise Diglycidylether von Bisphenol A, Epoxy-Phenol-Novolake, Diglycidylether von 1,4-Butandiol, Epoxy-Kresol-Novolake, Triglycidyl-p-aminophenol, Triglycidyl-tris-(p-hydroxyophenyl)-methan, Tetraglycidyl-1,1,2,2-tetrakis-(p-hydroxyphenyl)ethan, Vinylcyclohexen-dioxid, N,N,N',N'-Tetraglycidyl-4,4'-methylen-bis-benzolamin, N,N,N',N'-Tetraglycidyl-m-xyloediamin, Diglycidylanilin, Resorcin-diglycidyl-ether, die Diglycidylether von Katechol oder von Hydrochinon, Diglycidyl-o-toluidin, Diglycidyl-isophthalat, Epoxidharze auf der Basis von Bisphenol F und S, sowie N,N,N',N'-Tetraglycidyl-1,3-bis-amino-methylcyclohexan.

Die verschiedenen Ether sind gegebenenfalls an den einzelnen Phenylringen durch nicht reaktive Gruppen substituiert, wie beispielsweise durch Alkyl oder Halogen.

Zu den bevorzugten Epoxidverbindungen zählen a) solche der Formel II

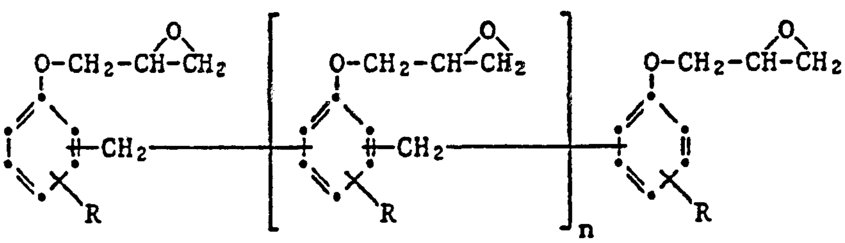

(II),

worin R Wasserstoff oder Methyl bedeutet und n 0,2—3,4 darstellt, insbesondere solche, worin R sich in o-position zur Glycidylethergruppe befindet.

Diese Komponenten sind als Epoxidierungsprodukte von Kresol-Novolaken oder von Phenol-Novolaken unterschiedlichen Molekulargewichtes bekannt. Die Herstellung solcher Verbindungen ist an sich bekannt.

Weitere bevorzugte Epoxidverbindungen sind b) solche der Formel III

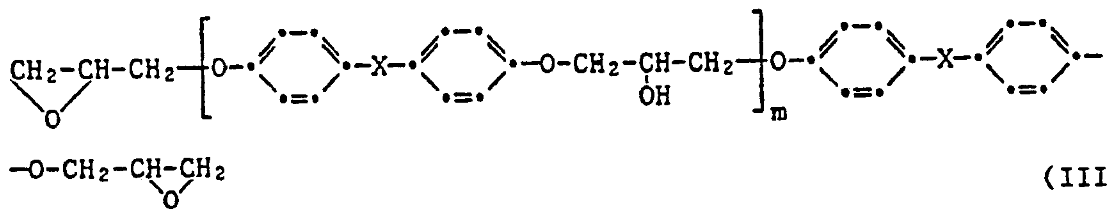

(III),

worin m 0—50 ist und X $—CH_2—$, $—C(CH_3)_2—$ oder $—SO_2—$ darstellt. Diese Diglycidylether leiten sich also von den Bisphenolen F, A und S ab.

Ebenfalls bevorzugt als Epoxidverbindung werden c) Diglycidylether von 1,4-Butandiol.

Die neuen Addukte der vorliegenden Erfindung werden in der Regel nach folgender Methode hergestellt.

Das Amin wird in einen Reaktionskessel gegeben und auf eine Temperatur von 80 bis 100°C erhitzt. Anschliessend wird das Polyepoxid zugefügt. Dies dauert in der Regel 60 bis 180 Minuten. Die Zugabegeschwindigkeit wird bevorzugt so eingeregelt, dass die maximale Reaktionshitze 125°C nicht überschreitet. Das Polyepoxid wird vorzugsweise in vorgewärmten Zustand zugefügt.

Nach Beendigung der Polyepoxidzugabe wird die Reaktionsmischung in der Regel 2 bis 6 Stunden lang auf einer Temperatur von 80 bis 125°C gehalten, um die Vollständigkeit der Umsetzung zu gewährleisten. Der Reaktionsverlauf kann durch Titration des Epoxidgruppengehaltes verfolgt werden. Dazu werden von Zeit zu Zeit Proben aus dem Reaktionsgemisch entnommen. Die vollständige Umsetzung ist erreicht, wenn keine Epoxidgruppen mehr nachweisbar sind.

Die so gewonnenen Addukte sind bei Raumtemperatur flüssig und besitzen eine mittlere Viskosität.

Bei der Herstellung dieser Addukte wird das Diamin im Ueberschuss von 2 bis 20 Molen, bevorzugt 2

bis 10 Molen, pro Mol Polyepoxid verwendet. Vorzugsweise setzt man 4 bis 8 Mole Diamin pro Mol Polyepoxid um, ganz besonders bevorzugt jedoch 6 Mole Diamin pro Mol Polyepoxid.

Den erfindungsgemässen Addukten können gegebenenfalls weitere Amine zugefügt werden. Damit lassen sich Härter herstellen, die den gehärteten Endprudukten spezielle Eigenschaften verleihen. So eignen sich in der Regel aliphatische, cycloaliphatische oder aromatische primäre oder sekundäre Amine. Bevorzugt sind die aliphatischen und cycloaliphatischen Amine.

Zu den typischen Verbindungen zählen beispielsweise Monoethanaolamin, N-Aminoethylethanolamin, Ethylendiamin, Hexamethylendiamin, Trimethylhexamethylendiamin, Diethylentriamin, Triethylentetraamin, Tetraethylenpentamin, N,N-Dimethylpropylen-1,3-diamin, N,N-Diethylpropylen-1,3-diamin, Bis(4-amino-3-methylcyclohexyl)methan, Bis(p-aminocyclohexyl)-methan, 2,2-Bis-(4-aminocyclohexyl)-propan, 3,5,5-Trimethyl-s-(aminomethyl)-cyclohexylamin, N-Aminoethylpiperazin, m-Phenylendiamin, p-Phenylendiamin, Bis(p-aminophenyl)-methan, Bis-(p-aminophenyl)-sulfon, m-Xylylendiamin, Toluoldiamin, 1,2-Diaminocyclohexan, 1,4-Diaminocyclohexan, 1,3-Bis-(aminomethyl)-cyclohexan, 1,4-Bis-(aminomethyl)-cyclohexan, Isophorondiamin und 1-Methyl-imidazol. Bevorzugte Amine sind Diethylentriamin, Triethylentetramin, Tetraethylenpentamin, 1,2-Diaminocyclohexan, Bis-(p-aminocyclohexyl)-methan, m-Xylyldendiamin, Isophorondiamin, 1,4-Bis-(aminomethyl)-cyclohexan, N-Aminoethyl-piperazin, 1,3-Bis-(aminomethyl)-cyclohexan, Bis-(p-aminophenyl)-methan und 1-Methylimidazol.

Diese Amine ligen in einer Maximalkonzentration von bis zu 75 Gew.%, bezogen auf die gesamte Härterzusammensetzung, vor. Ihr maximaler Anteil beträgt vorzugsweise bis zu 55 Gew.%.

Wie bereits weiter oben geschildert, können die modifizierten Härtersysteme in Kombination mit einer grossen Anzahl von Epoxidharzen verarbeitet werden.

Zu diesen Epoxidharzen zählen beispielsweise solche, die sich von mehrwertigen Phenolen ableiten, beispielsweise von Bisphenol A, F, und S, sowie Epoxidierungsprodukte von Kresol-Novolaken und Phenol-Novolaken; ferner zählen dazu Epoxidharze auf Hydantoinbasis, Polyglycidylester, glycidylierte aromatische Amine, glycidylierte Aminophenole und gewisse cycloaliphatische Epoxidharze.

Bei Verwendung als Klebstoff oder Beschichtungsmittel sowie beim Filament-Winding werden in der Regel Diglycidylether auf Basis von Bisphenol A eingesetzt.

Der modifizierte Härter wird in stöchiometrischen Mengen bzw. in Abweichung von + oder −50% der stöchiometrischen Menge, bezogen auf das Epoxidharz eingesetzt. Bevorzugt wird eine Härtermenge im Bereich von ± 15% der stöchiometrischen Menge verwendet, bezogen auf das Epoxidharz.

Abgesehen von den oben beschriebenen Anwendungsgebieten, sind die Addukte dieser Erfindung nützliche Härter für eine weitere Anzahl von Epoxidharzen in verschiedenen Anwendungsgebieten, bei denen wärmegehärtete Produkte eingesetzt werden.

Wenn die Härter dieser Erfindung beispielsweise mit Di- und Polyepoxiden in üblicherweise stöchiometrischen Mengen kombiniert werden und bei erhöhten Temperaturen gehärtet werden, so entsteht ein Netzwerk mit hoher Vernetzungsdichte.

Der Ausdruck 'Härten', so wie er hier gebraucht wird, bedeutet die Ueberführung der obenerwähnten Addukte und Epoxidmaterialien in unlösliche und nicht schmelzbare vernetzte Produkte, unter gleichzeitiger Formgebung, wobei geformte Artikel entstehen, beispielsweise Giesslinge, Presslinge oder Laminate, oder wobei zweidimensionale Strukturen entstehen, wie beispielsweise Beschichtungen, Lacke oder Klebverbindungen.

Besonders bevorzugt setzt man das modifizierte Härtersystem bei der Bildung von Beschichtungen ein, da es sich gut mit Harzen verträgt und da die resultierenden gehärteten Ueberzüge eine verbesserte Zähigkeit aufweisen.

Die erfindungsgemäss hergestellten Addukte, die sich mit anderen Polyepoxiden mischen lassen, können darüber hinaus auf jeder Stufe vor der Härtung noch mit weiteren üblichen Zusätzen versehen werden, wie beispielsweise mit Streckmitteln, Füllstoffen und Verstärkungsmitteln, Pigmenten, Farbstoffen, organischen Lösungsmitteln, Weichmachern, Mitteln zur Beeinflussing der Klebrigkeit, Kautschuken, Härtungsbeschleunigern oder Verdünnungsmitteln.

Zu den Streckmitteln, Verstärkungsmitteln, Füllstoffen und Pigmenten, die in den erfindungsgemässen härtbaren Zusammensetzungen verwendet werden können, zählen beispielsweise Kohleteer, Bitumen, Glasfasern, Borfasern, Kohlefasern, Cellulose, Polyethylenpulver, Polypropylenpulver, Glimmer, Asbest, Quarzmehl, Gips, Antimontrioxid, Bentone, Talkum, Kieselgel (Aerosil®), Lithopone, Baryt, Calziumcarbonat, Titandioxid, Russ, Graphit, Eisenoxid oder Metallpulver, wie beispielsweise Aluminiumpulver oder Eisenpulver.

Es können darüber hinaus noch weitere übliche Additive den erfindungsgemässen Zusammensetzungen zugesetzt werden. Beispiele dafür sind Mittel zur Erhöhung der Flammfestigkeit, Thixotropiemittel, Mittel zur Kontrolle der Fliesseigenschaften, beispielsweise Silikone, Cellulosacetatbutyrat, Polyvinylbutyral, Wachse oder Stearate. Solche Zusätze werden teilsweise als Entformungsmittel verwendet. Gegebenenfalls verwendete Beschleuniger dienen zur Erhöhung der Härtungsgeschwindigkeit bei der Herstellung von dünnen Filmen, d.h., bei Filmen mit einer maximalen Dicke von 200 µm pro Schicht.

Typische Beschleuniger sind beispielsweise aromatische Carbonsäuren, wie Benzosäure und Salicylsäure; Phenole, wie Phenol, p-tert.Butyl-phenyl, Bisphenol A und Nonylphenol; oder aromatische Alkohole, wie Benzylalkohol.

Gegebenenfalls eingesetzte Lösungsmittel modifizieren die härtbaren Mischungen; sie dienen

insbesondere zur Viskositätskontrolle. Als Lösungsmittel lassen sich beispielsweise Etheralkohole einsetzen, wie Ethylenglykol-monomethylether, -monoethylether, -monobutylether, sowie die entsprechendne Diethylenglykol-Analogen; oder es lassen sich aromatische Kohlenwasserstoffe verwenden, wie beispielsweise Xylol und Toluol. Ferner ist es möglich, beispielsweise bei Klebstofformulierungen, Kautschuke zuzusetzen, beispielsweise carboxylterminierte Acrylnitril-Butadien Kautschuke, oder modifizierende Harze, wie beispeilsweise Triglycidyl-p-aminophenol, oder Beschleuniger, wie beispielsweise Komplexe von Bortrifluorid mit Monoethylamin oder mit Imidazolen. Ferner können noch weitere Härter anwesend sein, wie beispielsweise Dicyandiamid.

Die härtbaren Zusammensetzungen können in der üblichben Art und Weise hergestellt werden, z.B. durch Verarbeitung in herkömmlichen Mischern (Rührer, Kneter, Roller).

Die härtbaren Epoxidzusammensetzungen sind besonders nützlich auf dem Gebiet des Oberflächenschutzes, in der elektrischen Industrie, bei Laminierverfahren und in der Bauindustrie.

Sie können als Formulierungen eingesetzt werden, die von Fall zu Fall an den vorgesehenen Verwendungszweck angepasst sind, in gefüllter oder ungefüllter Form, gegebenenfalls als Lösungen oder Emulsionen, als Anstrichfarben, Sinterpulver, Pressmassen, Tauchharze, Giessharze, Spritzgussformulierungen, Imprägnierharze und Klebstoffe, als Werkstoffharze, Laminierharze, Verguss- und Füllstoffmassen, als Fussbodendeckungen oder als Bindemittel für mineralische Aggreate.

Besonderes Interesse verdient ihr Einsatz bei der Herstellung von wärmegehärteten Laminaten, wobei die oben erwähnte Eigenschaft der Rückkehr der Fliessfähigkeit zur Erzielung optimaler Eigenschaften des Endproduktes ausgenutzt werden kann.

Die Addukte der vorliegenden Erfindung können ebenso zum thermischen Aushärten von Polyurethanharzen verwendet werden.

Dazu vermischt man diese Härter nach an sich bekannten Techniken mit dem Harz. Die Polyisocyanate, die in den erfindungsgemässen härtbaren Polyurethanmassen eingesetzt werden können, entsprechen denjenigen, die üblicherweise in der Urethanharzherstellung Anwendung finden; beispielsweise gehören dazu: Toluoldiisocyanat, 4,4-Diphenylmethan-diisocyanat, Polyarylpolyisocyanate oder Hexamethylendiisocyanat, oder auch weniger gängige Ausgansmaterialien, wie beispielsweise Phenylindandiisocyanat.

Bekanntermassen sind Harze, die sich von solche Isocyanaten ableiten, spröde, so dass es in den meisten Fällen angebracht und bevorzugt ist, ein modifiziertes Präpolymers mit durchschnittlich mehr als einer Isocyanatgruppe pro Molekül einzusetzen.

Das konventionelle Präpolymere eines Polyisocyanates mit durchschittlich mehr als einer Isocyanatgruppe pro Molekül leitet sich üblicherweise von einen der oben erwähnten Diisocyanate ab, das in molarem Ueberschuss mit einer Verbindung enthaltend mindestens zwei Hydroxylgruppen im Molekül und mit einem Molekulargewicht von wenigstens 300, beispielsweise mit Rizinusöl, einem hydroxyterminierten Polyether, einem hydroxy-terminierten Polyester, einem hydroxy-terminierten Polybutadien oder -Butadien-Acrylnitril Copolymeren umgesetzt wird.

Als hydroxy-terminierter Polyether wird beispielsweise ein Polyalkylenglykol verwendet, worin jede Alkylengruppe zwischen 2 und 6 Kohlenstoffatomen enthält.

Als hydroxy-terminierter Polyester wird vorzugsweise ein aliphatischer Polyester eingesetzt, der sich ableitet von einem $C_2$—$C_6$Alkylkenglykol und einer aliphatischen Polycarbonsäure, die neben den Carboxylgruppen lediglich Kohlenwasserstoffgruppen enthält; die bevorzugte Kohlenstoffanzahl dieser Säure beträgt zwischen 3 und 10.

Polyether, wie beispielsweise Polyethylenglykol, Polypropylenglykol oder Polytetramethylenglykol mit Molekulargewichten zwischen 300 und 2000 werden bevorzugt, ebenso werden die hydroxy-terminierten Polyester beliebiger Polyalkylenglykole bevorzugt, insbesondere diejenigen, die sich von $C_2$—$C_6$Alkylenglykolen mit Polycarbonsäuren enthaltend 3—10 Kohlenstoffatome und enthaltend ausser den Carboxylgruppen nur noch Kohlenwasserstoffreste ableiten. Diese bevorzugten Polyester besitzen mittlere Aequivalentgewichte von 150—1000 (bezogen auf die Hydroxylgruppen) und haben 2 bis 4 Hydroxylgruppen pro Molekül.

Bevorzugt werden diejenigen Präpolymeren, die durch Umsetzung von wenigstens zwei molaren Anteilen eines Diisocyanates, wie oben erwähnt, mit einem Polyalkylenglykol, wie oben erwähnt, erhalten werden, wobei ein Präpolymer entsteht, das ein Aequivalentgewicht von 400—1500 (bezogen auf die Isocyanatgruppen) besitzt. Es können jedoch auch andere Präpolymere mit einem Aequivalentgewicht (bezogen aud die Isocyanatgruppen) wie oben definiert eingesetzt werden. Ebenso kann man partiell blockierte Polyisocyanate verwenden.

Kombiniert man die erfindungsgemässen Addukte mit Polyurethanen in üblicherweise stöchiometrischen Mengen und härtet diese bei erhöhten Temperaturen, so entsteht beim Endprodukt das bereits weiter oben für die Epoxidharze erwähnte Netzwerk hoher Verknüpfungsdichte.

Diese Polyurethansysteme können gegebenenfalls noch mit weiteren unterschieldlichen Zusatzstoffen vermischt werden. Beispiele für solche Zusatzstoffe wurden schon weiter oben für Epoxidharzsysteme aufgeführt; insbesondere sind hier noch spezielle Polyurethanstreckmittel und -weichmacher zu erwähnen.

In den folgenden Beispielen bedeuten Teile Gewichsteile.

Beispiel 1

Dieses Beispiel illustriert die Herstellung eines typischen modifizierten Härters der vorliegenden

Erfindung. Es wird ein Addukt aus Diethyltoluoldiamin und Diglycidylether von Bisphenol A hergestellt, wobei das molare Verhältnis 4:1 beträgt. 531 g Diethyltoluoldiamin (76% 2,4-Diethylisomer und 19% 4,6-Diethylisomer; ETHACURE® 100 der Ethyl Corp.) werden in einen Reaktionskolben eingewogen, der mit mechanischem Rührer, Thermometer und Heizmantel ausgestattet ist. Der Kolbeninhalt wird unter Rühren auf 80°C erhitzt. 269 g vorgewärmten Diglycidylethers von Bisphenol A mit einem Epoxidgehalt von 5,1—5,5 Aequ./kg werden durch einen Tropftrichter hinzugegeben. Die Temperatur wird für 2 Stunden bei 80°C belassen (keine exotherme Reaktion). Anschliessend wird die Temperatur auf 100°C gesteigert und die einsetzende exotherme Reaktion lässt die Temperatur für eine Stunde auf 125°C steigen. Man erhält ein Addukt mit einem mittleren Molekulargewicht im Bereich von 720—740.

Beispiel 2

Es wird ein Addukt aus Diethyltoluoldiamin wie in Beispiel 1 und einem Epoxiphenol-Novolak hergestellt, indem man die Komponenten in einem molaren Verhältnis von 6:1 umsetzt. Dazu werden 680 g Diethyltoluoldiamin in einen Reaktionskolben eingewogen, der mit mechanischem Rührer, Thermometer und Heizmantel ausgestattet ist. Der Kolbeninhalt wird unter Rühren auf 100°C erhitzt. 120 g des vorgewärmten Epoxiphenol-Novolakharzes mit einem Epoxidgehalt von 5,5—5,7 Aequ./kg werden langsam unter Rühren zugegeben. Nach beendeter Zugabe wird die Temperatur 3 Stunden lang bei 100°C belassen, um die Reaktion zu vervollständigen.

Man erhält ein Addukt dessen IR-Spektrum eine maximale Absorption bei 3370, 2957, 2891, 1625, 1478 und 1451 $cm^{-1}$ aufweist. Das Produkt besitzt einen Erweichungspunkt bei −21°C.

Beispiele 3 und 4

Das Verfahren von Beispiel 1 wird wiederholt, indem man die folgenden Komponenten miteinander umsetzt

| | Teile | |
|---|---|---|
| | Beispiel 3 | Beispiel 4 |
| Diglycidylether von Bisphenol A [1)] | 28,1 | — |
| 1,4-Butandiol-diglycidylether | — | 18,5 |
| Diethyltoluoldiamin (wie in Beispiel 1) | 71,9 | 81,5 |

1) DER® 332 der Dow (Epoxidgehalt: 5,7 Aequ./kg)

Die nachfolgenden charakteristischen Daten werden für die Addukte der Beispiele 3 und 4 gemessen:

| | Beispiel 3 | Beispiel 4 |
|---|---|---|
| Erweichungspunkt (°C) | −15 | −20 |
| Maximale IR Absorption ($cm^{-1}$) | 3380, 2957, 2891, 1625, 1478, 1451 | 3370, 2957, 2891, 1625, 1478, 1451 |

Beispiel 5

Das folgende Beispiel beschreibt die physikalischen Eigenschaften und die Gebrauchseigenschaften von Epoxidharzen, die unter Verwendung der Härter aus Beispielen 1 bis 4 verarbeitet werden.

Die Harzsysteme werden hergestellt, indem man die Harzkomponente zusammen mit allfälligen Zusätzen und dem betreffenden Härter bei Raumtemperatur mischt.

Für die Formulierung A wird ein Epoxidharz eingesetzt, das aus 65 Teilen Diglycidylether von Bisphenol A (DER® 232), 25 Teilen Epoxyphenol-Novolak (wie in Beispiel 2 verwendet) und 10 Teilen 1,4-Butandioldiglycidylether besteht.

Für die Formulierungen B bis G wird ein Epoixharz eingesetzt, das mit 45 Teilen Eisenoxid gefüllt ist und 47 Teile Diglycidylether von Bisphenol A (DER® 332), sowie 3 Teile, 1,4-Butandioldiglycidylether enthält.

Folgende Eigenschaften werden bestimmt:

*Viskosität der Mischung:* Die Viskosität der Epoxidharz-Härter Mischung wird mittels eines Brookfield RVF Viskosimeters bestimmt. Dazu wird die Spindel # 3 verwendet. Gemessen wird bei 20 Umdrehungen pro Minute und bei 23—25°C. Die Proben werden 2 Minuten gemischt und die Viskositätsbestimmung erfolgt eine Minute nach der Mischung.

*Wärmeformbeständdigkeit:* Die Bestimmung erfolgt gemäss ASTM D-648-82. Das flüssige Material wird in eine Gussform gegeben und über Nacht bei Raumtemperatur gehärtet. Anschliessend wird gemäss dem angegebenen Härtungszyklus (s. Tabelle) gehärtet. Der Test wird mit Probeplatten der Ausmasse 1,27 cm × 1,27 cm × 1,27 cm unter Anwendung eines Druckes von 18,2 bar durchgeführt.

*Topfzeit:* Die unten angegebenen Mengen des Epoxidharz/Härter Gemisches werden bei Raumtemperatur in einem Metallgefäss reagieren gelassen. Die Zeit bis zum vollständigen Umsatz wird gemessen. Die Mischung wird als durchgehärtet angesehen, wenn sie bei Raumtemperatur fest ist.

*Klebrigkeit einer dünnen Schicht:* Dieser Test ein Mass für diejenige Zeit, die benötigt wird, um ein Harz-/Härtersystem genügend auszuhärten, so dass eine feste Form entsteht, die einen Fingerabdruck an der Oberfläche des Films sichtbar bleiben lässt. Zur Durchführung des Tests trägt man eine 0,5 mm dicke Schicht des Epoxidsystems auf eine Oberfläche auf (bei Raumtemperatur) und erzeugt zu definierten Zeitabständen einen Fingerabruck auf der Oberfläche.

Die Zusammensetzungen der getesteten Formulierungen finden sich in der folgenden Tabelle Ia, während die Testergebnisse in der Tabelle Ib aufgelistet sind.

Tabelle Ia:

| | Teile | | | | | | |
|---|---|---|---|---|---|---|---|
| Formulierung | A | B | C | D | E | F | G |
| Addukt von Beispiel 1 | 45 | - | - | - | - | - | - |
| Addukt von Beispiel 2 | - | 35 | 28 | 85 | 90 | - | - |
| Addukt von Beispiel 3 | - | 50 | 39 | - | - | 50 | 50 |
| Addukt von Beispiel 4 | - | - | 9 | - | - | - | 35 |
| Triethylentetramin | 50 | - | - | - | - | - | - |
| Diethyltoluoldiamin | - | - | 9 | - | - | 35 | - |
| m-Xyloldiamin | - | 15 | 15 | 15 | 10 | 15 | 15 |
| 1-Methylimidazol | 5 | - | - | - | - | - | - |
| Verhältnis modifiziertes Epoxidharz zu Härter | 100/13 | 100/17 | 100/16 | 100/13 | 100/15 | 100/16 | 100/17 |

Tabelle Ib:

| Formulierung | A | B | C | D | E | F | G |
|---|---|---|---|---|---|---|---|
| Viskosität der Mischung (cps) | 1660 | | | | | | |
| Wärmeformbeständigkeit (°C) | 141 | 146 | 146 | 148 | 152 | 145 | 143 |
| Härtungszyklus *) | 1 | 2 | 2 | 2 | 2 | 2 | 2 |
| Topfzeit (Gesamtmenge 175 g) (Min.) | 65 | - | - | - | - | - | - |
| Topfzeit (Gesamtmenge 210 g) (Min.) | - | - | - | 35 | 50 | - | 20 |
| Klebrigkeit einer dünnen Schicht (h) | - | - | - | 16 | 20 | - | - |

*) Härtungszyklen

Zyklus 1: 2 h bei 65°C; 1 h bei 95°C; 1 h bei 150°C; 3 h bei 170°C

Zyklus 2: 2 h bei 65°C; 2 h bei 95°C; 2 h bei 120°C; 2 h bei 150°C; 4 h bei 170°C

Beispiel 6

Ein gehärtetes Polyurethansystem wird hergestellt, indem man bei Raumtemperatur 24 Teile des Adduktes von Beispiel 1, 24 Teile eines Weichmachers auf der Basis von Diotylphthalat und 100 Teile eines cycloaliphatischen Isocyanat-Urethansystems mit einem Isocyanatgehalt von 2,3—2,4 Aequ./kg mischt.

Nach Aushärtung bei erhöhter Temperatur besitzt das feste Urethanharz einen Shore A Härtewert von 90—95.

**Patentansprüche**

1. Addukt erhältlich durch Umsetzung von Diethyltoluoldiamin mit einem Polyepoxid, das eine Funktionalität von wenigstens zwei aufweist, wobei besagtes Diamin bezogen auf besagtes Polyepoxid im Verhältnis von 2—20 Molen Diamin pro Mol Polyepoxid eingesetzt wird.

2. Härter gemäss Anspruch 1, worin besagtes Polyepoxid ausgewählt wird aus der Gruppe bestehend aus Diglycidylethern von Bisphenol A, Epoxy-Phenol-Novolaken, Diglycidylether von 1,4-Butandiol, Epoxy-Kresol-Novolaken, Triglycidyl-p-aminophenol, Triglycidyl-tris-(p-hydroxyphenyl)-methan, Tetraglycidyl-1,1,2,2-tetrakis-(p-hydroxyphenyl)-ethan, Vinylcyclohexendioxid, N,N,N[1],N[1]-Tetraglycidyl-4,4[1]-methylen-bis-benzolamin, N,N,N[1],N[1]-Tetraglycidyl-m-xyloldiamin, Diglycidylanilin, Diglycidylethern von Resorcin, die Diglycidylethern von Katechol, Diglycidylethern von Hydrochinon, Diglycidyl-o-toluidin, Diglycidyl-isophthalat, Epoxidharzen auf Bisphenol F und Bisphenol S Basis, sowie N,N,N[1],N[1]-Tetraglycidyl-1,3-bis-amino-methylcyclohexan.

3. Härter gemäss Anspruch 1, worin besagtes Polyepoxid eine Verbindung der Formel II darstellt

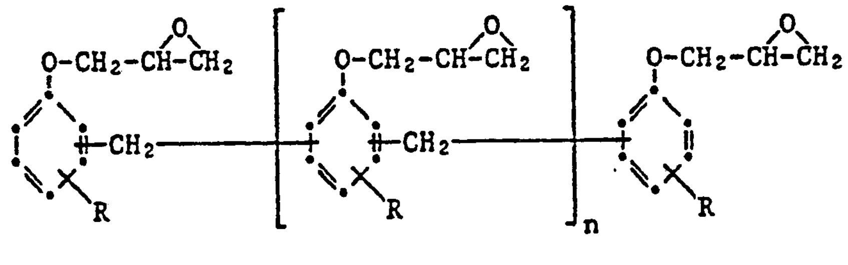

(II),

worin R Wasserstoff oder Methyl ist, die Methylgruppe sich vorzugsweise in o-Position zur Glycidylethergruppe befindet, und n 0,2—3,4 bedeutet.

4. Härter gemäss Anspruch 1 worin besagtes Polyepoxid eine Verbindung der Formel III darstellt

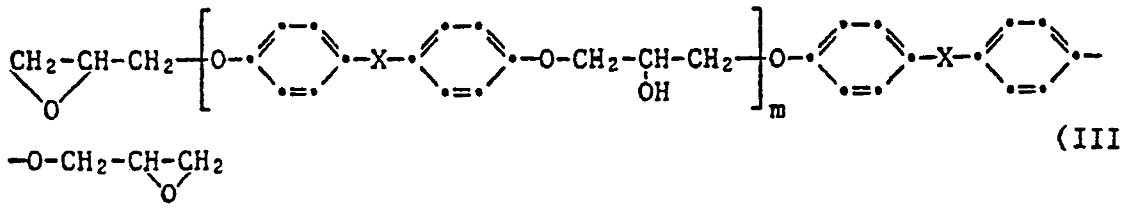

(III),

worin m 0—50 ist und X $-CH_2-$, $-C(CH_3)_2-$ oder $-SO_2-$ bedeutet.

5. Härter gemäss Anspruch 1, worin besagtes Polyepoxid ein Diglycidylether von 1,4-Butandiol ist.

6. Härtbare Zusammensetzung enthaltend a) eine Polyepoxidverbindung und b) ein Addukt gemäss Anspruch 1.

7. Härtbare Zusammensetzung gemäss Anspruch 6, worin besagte Polyepoxidverbindung ausgewählt ist aus der Gruppe bestehend aus Epoxidharzen auf der Basis mehrwertiger Alkohole, Epoxidierungsprodukten von Kresol-Novolaken, Epoxidierungsprodukten von Phenol-Novolaken, Epoxidharzen, auf Hydantoinbasis, Polyglycidylestern, glycidylierten aromatischen Aminen, glycidylierten Aminophenolen und cycloaliphatischen Epoxidharzen.

8. Härtbare Zusammensetzung gemäss Anspruch 6, worin besagte Polyepoxidverbindung und besagter Härter in Mengen von ±50% des stöchiometrischen Verhältnisses vorhandedn sind.

9. Härtbare Zusammensetzung gemäss Anspruch 6, die zusätzlich bis zu 75 Gew.% bezogen auf den Härter, eines aliphatischen, cycloaliphatischen oder aromatischen primären oder sekundären Amins enthält.

10. Härtbare Zusammensetzung gemäss Anspruch 9, worin besagtes Amin ausgewählt ist aus der Gruppe bestehend aus Monoethanolamin, N-Aminoethylethanolamin, Ethylendiamin, Hexamethylendiamin, Trimethylhexamethylendiamin, Diethylentriamin, Triethylentetraamin, Tetraethylenpentamin, N,N-Dimethylpropylendiamin-1,3, N,N-Diethylpropylendiamin-1,3, Bis-(4-amino-3-methylcyclohexyl)-methan, Bis(p-aminocyclohexyl)-methan, 2,2-Bis-(4-aminocyclohexyl)-propan, 3,5,5-Trimethyl-s-(aminomethyl)-cyclohexylamin, N-Aminoethylpiperazin, m-Phenylendiamin, p-Phenylendiamin, Bis-(p-aminophenyl)-methan, Bis-(p-aminophenyl)-sulfon, m-Xylylendiamin, 1,2-Diaminocyclohexan, 1,4-Diaminocyclohexan, Toluoldiamin, 1,3-Bis-(aminomethyl)-cyclohexan, 1,4-Bis-(aminomethyl)-cyclohexan, Isophorondiamin und 1-Methyl-imidazol.

11. Härtbare Zusammensetzung gemäss Anspruch 6, worin in besagtem Addsukt b) besagtes Polyepoxid der Formel II entspricht

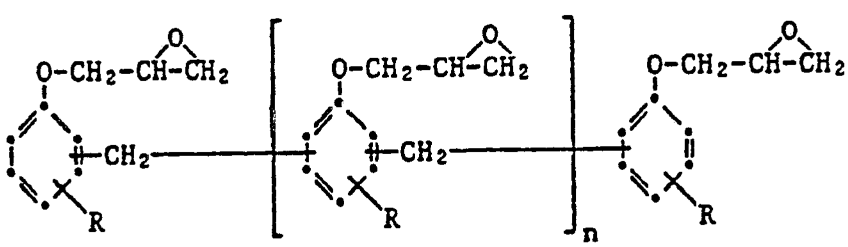

(II),

worin R Wasserstoff oder Methyl ist, die Methylgruppe sich vorzugsweise in o-Position zur Glycidylethergruppe befindet, und n 0,2—3,4 bedeutet.

12. Härtbare Zusammensetzung gemäss Anspruch 6, worin in besagtem Addukt b) besagtes Polyepoxid der Formel III entspricht

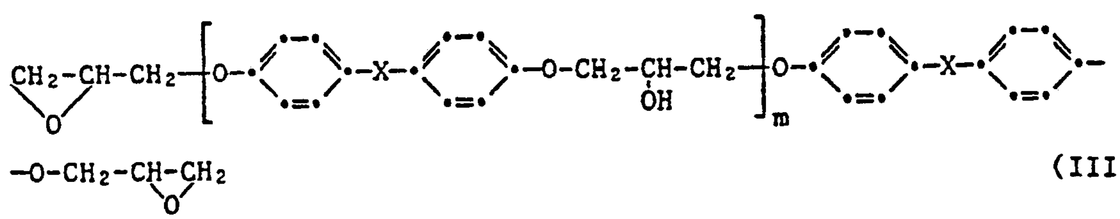

(III),

worin m 0—50 ist und X $-CH_2-$, $-C(CH_3)_2-$, oder $-SO_2-$ darstellt.

13. Härtbare Zusammensetzung gemäss Anspruch 6, worin in besagtem Addukt b) besagtes Polyepoxid ein Diglycidylether des 1,4-Butadiols ist.

14. Härtbare Zusammensetzung gemäss Anspruch 6, worin in besagtem Addukt b) 2—20 Mole Diamin

pro Mol Polyepoxid eingesetzt werden.

15. Das gehärtete Produkt erhältlich durch Aushärtung einer Zusammensetzung gemäss Anspruch 6 bei erhöhten Temperaturen.

16. Das gehärtete Produkt erhältlich durch Aushärtung einer Zusammensetzung gemäss Anspruch 9 bei erhöhten Temperaturen.

17. Härtbare Zusammensetzung enthaltend a) ein Urethanharz und b) ein Addukt gemäss Anspruch 1.

18. Das gehärtete Produkt erhältlich durch Aushärtung von Zusammensetszungen gemäss Anspruch 17 bei erhöhten Temperaturen.

**Revendications**

1. Produits d'addition obtenus par réaction de la diéthyloluène-diamine avec un polyépoxyde ayant un degré de fonctioonnalité au moins égal à 2, dans des proportions de 2 à 20 moles de la diamine par mole du polyépoxyde.

2. Durcisseurs selon la revendication 1 pour lesquels le polyépoxyde est pris parmi les suivants: éthers diglycidyliques du Bisphénol A, novolaques époxyphénol, éthers diglycidyliques du 1,4-butane-diol, novolaques époxy-crésol, triglycidyl-p-amino-phénol, triglycidyl-tris-(p-hydroxyphényl)-méthane, tétraglydicyl-1,1,2,2-tétrakis-(p-hydroxyphényl)-éthane, dioxyde de vinylcyclohexène, N,N,N$^1$,N$^1$-tétraglycidyl-4,4$^1$-méthylène-bis-benzolamine, N,N,N$^1$,N$^1$-tétraglydicyl-m-xylène-diamine, diglycidylaniline, éthers diglycidyliques de résorcinol, de catéchol ou d'hydroquinone, diglycidyl-o-toluidine, isophtalate de diglycidyle, résines époxydes à base de Bisphénol F et de Bisphénol S et N,N,N$^1$,N $^1$-tétraglydicyl-1,3-bis-amino-cyclohexane.

3. Durcisseurs selon la revendication 1 pour lesquels le polyépoxyde est un composé de formule II:

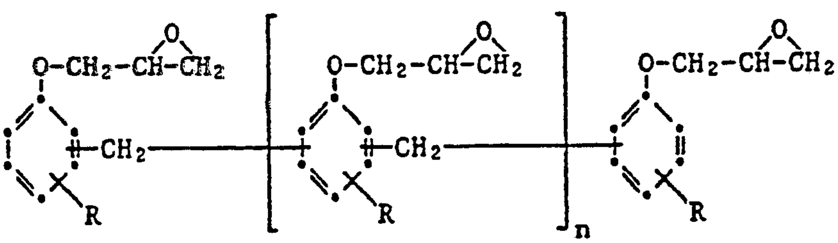

(II),

dans laquelle R désigne l'hydrogène ou le groupe méthyle, s'il s'agit du groupe méthyle de préference à une position ortho par rapport au groupe d'éther glycidylique, et n est un nombre de 0,2 à 3,4.

4. Durcisseurs selon la revendication 1 pour lesquels le polyépoxyde est une compose de formule III:

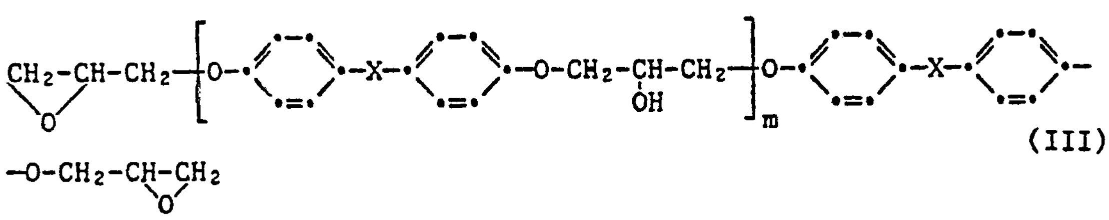

(III),

m étant un nombre de 0 à 50 et X le groupe $—CH_2—$, $—C(CH_3)_2—$ ou $—SO_2—$.

5. Durcisseur selon la revendication 1 pour lequel le polyépoxyde est un éther diglycidyliques du 1,4-butane-diol.

6. Compositions durcissables comprenant a) un composé polyépoxydique et b) un produit d'addition selon la revendication 1.

7. Compositions durcissables selon la revendication 6 dans lesquelles le composé polyépoxydique est pris parmi des résines époxydes à base de polyols, des produits d'époxydation de novolaques de crésol ou de novolaques de phénol, des résines époxydes à base d'hydantoïne, des esters polyglycidyliques, des amines aromatiques glycidylées, des aminophénols glycidylés et des résines époxydes cycloaliphatiques.

8. Compositions durcissables selon la revendication 6 dans lesquelles le composé polyépoxydique et le durcisseur se trouvent dans des proportions qui peuvent sécarter de plus ou moins 50% du rapport stoéchiométrique.

9. Compositions durcissables selon la revendication 6 qui contiennent en outre jusqu'à 75%, du poids du durcisseur, d'une amine aliphatique, cycloaliphatique ou aromatique, primaire ou secondaire.

10. Compositions durcissables selon la revendication 9 dans lesquelles l'amine est prise parmi les suivantes: monoéthanolamine, N-amino-éthyl-éthanolamine, éthylène-diamine, hexaméthylène-diamine,

triméthylhexaméthylène-diamine, diéthylène-triamine, triéthylène-tétramine, tétraéthylène-pentamine, N,N-diméthyl-propylène-diamine-1,3, N,N-diéthyl-propylène-diamine-1,3, bis-(4-amino-3-méthylcyclohexyl)-méthane, bis-(p-aminocyclohexyl)-méthane, 2,2-bis-(4-aminocyclohexyl)-propane, 3,5,5-triméthyl-s-(aminométhyl)-cyclohexylamine, N-aminoéthyl-pipérazine, m-phénylène-diamine, p-phénylène-diamine, bis-(p-aminophényl)-méthane, bis-(p-aminophényl)-sulfone, m-xylylène-diamine, 1,2-diaminocyclohexane, 1,4-diaminocyclohexane, toluène-diamine, 1,3-bis-(aminométhyl)-cyclohexane, 1,4-bis-(aminométhyl)-cyclohexane, isophorone-diamine et 1-méthyl-imidazole.

11. Compositions durcissables selon la revendication 6 dans lesquelles le polyépoxyde du proudit d'addition b) est un composé de formule II:

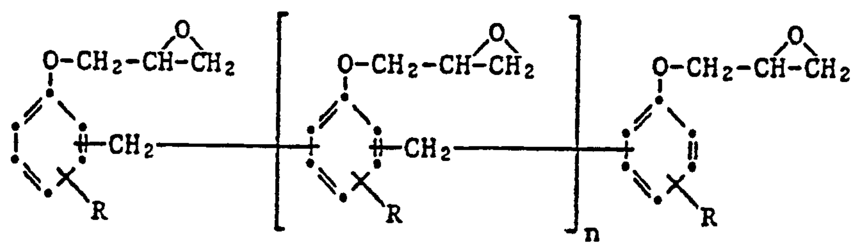

(II),

dans lquelle R désigne l'hydrogéne ou le groupe méthyle, s'il s'agit du groupe méthyle de préférence à une position ortho par rapport au groupe d'éther glycidylique, et n est un nombre de 0,2 à 3,4.

12. Compositions durcissables selon la revendication 6 dans lesquelles le polyépoxyde du produit d'addition b; est un composé de formule III:

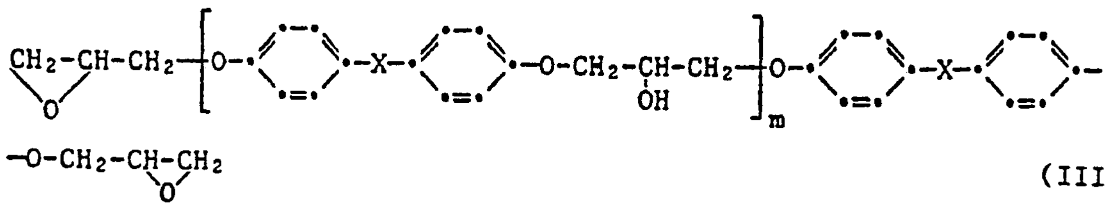

(III),

m étant un nombre de 0 à 50 et X le groupe $-CH_2-$, $-C(CH_3)_2-$ ou $-SO_2-$.

13. Compositions durcissables selon la revendication 6 dans lesquelles le polyépoxyde du produit d'addition b) est un éther diglycidylique du 1,4-butane-diol.

14. Compositions durcissables selon la revendication 6 dans lesquelles le produit d'addition b) a été formé avec de 2 à 20 moles de la diamine par mole du polyépoxyde.

15. Les produits durcis obtenus par durcissement à chaud d'une composition selon la revendication 6.

16. Les produits durcis obtenus par durcissement à chaud d'ujne composition selon la revendication 9.

17. Compositions durcissables comprenant a; une résine de polyuréthanne et b) un produit d'addition selon la revendication 1.

18. Les produits durcis obtenus par un durcissement à chaud d'une composition selon la revendication 17.

**Claims**

1. An adduct obtainable by reaction of diethyltoluenediamine with a polyepoxide having a functionality of at least two, said diamine being employed in the ratio of 2—20 moles of diamine per mole of polyepoxide, based on said polyepoxide.

2. A hardener according to claim 1, in which said polyepoxide is selected from the group consisting of diglycidyl ethers of bisphenol A, epoxy phenol novolacs, 1,4-butanediol diglycidyl ethers, epoxy cresol novolacs, triglycidyl-para-aminophenol, triglycidyl-tris(p-hydroxyphenyl)-methane, tetraglycidyl-1,1,2,2-tetrakis-(p-hydroxyphenyl)-ethane, vinyl cyclohexene dioxide, $N,N,N^1,N^1$-tetraglycidyl-4,4$^1$-methylene-bis benzeneamine, $N,N,N^1,N^1$-tetraglycidyl-meta-xylenediamine, diglycidylaniline, resorcinol diglycidyl ethers, catechol diglycidyl ethers, hydroquinone diglycidyl ethers, diglycidyl-ortho-toluidine, diglycidyl isophthalate, bisphenol F and S epoxy resins, and $N,N,N^1,N^1$-tetraglycidyl-1,3-bis-aminomethylcyclohexane.

3. A hardener according to claim 1, in which said polyepoxide is a compound of the formula II

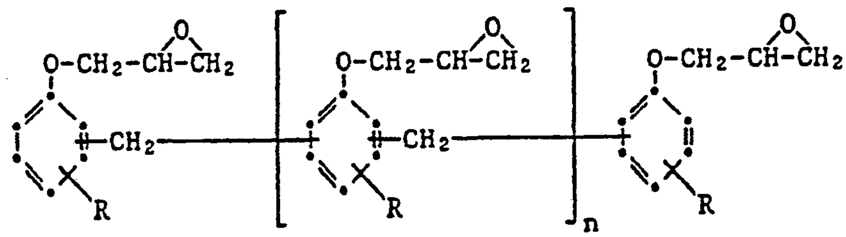

(II),

in which R is hydrogen or methyl, the methyl group is preferably in the ortho-position to the glycidyl ether group, and n is 0.2—3.4.

4. A hardener according to claim 1, in which said polyepoxide is a compound of the formula III

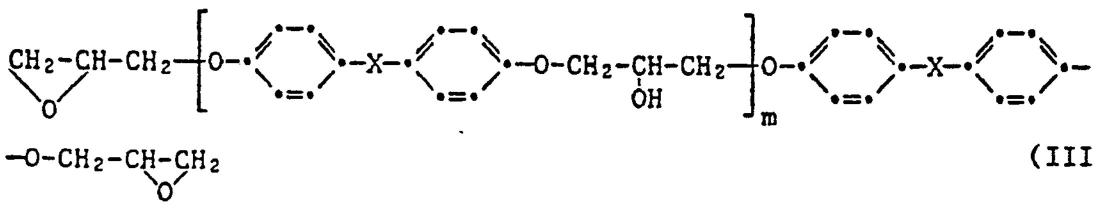

(III),

in which m is 0—50 and X is $—CH_2—$, $—C(CH_3)_2—$ or $—SO_2—$.

5. A hardener according to claim 1, in which said polyepoxide is a 1,4-butanediol diglycidyl ether.

6. A curable composition containing a) a polyepoxide compound and b) an adduct according to claim 1.

7. A curable composition according to claim 6, in which said polyepoxide compound is selected from the group consisting of epoxy resins based on polyhydric alcohols, epoxidation products of cresol novolacs, epoxidation products of phenol novolacs, hydantoin epoxy resins, polyglycidyl esters, glycidylated aromatic amines, glycidylated aminophenols and cycloaliphatic epoxy resins.

8. A curable composition according to claim 6, in which said polyepoxide compound and said hardener are present in amounts of ±50% of the stoichiometric ratio.

9. A curable composition according to claim 6, which additionally contains up to 75% by weight, based on the hardener, of an aliphatic, cycloaliphatic or aromatic primary or secondary amine.

10. A curable composition according to claim 9, in which said amine is selected from the group consisting of monoethanolamine, N-aminoethylethanolamine, ethylenediamine, hexamethylenediamine, trimethylhexamethylenediamine, diethylenetriamine, triethylenetetraamine, tetraethylenepentamine, N,N-dimethylpropylene-1,3-diamine, N,N-diethylpropylene-1,3-diamine, bis(4-amino-3-methylcyclohexyl)methane, bis(p-aminocyclohexyl)methane, 2,2-bis(4-aminocyclohexyl)propane, 3,5,5-trimethyl-s-(aminomethyl)-cyclohexylamine, N-aminoethylpiperazine, m-phenylenediamine, p-phenylenediamine, bis(p-aminophenyl)methane bis(p-aminophenyl)sulphone, m-xylylenediamine, 1,2-diaminocyclohexane, 1,4-diaminocyclohexane, toluenediamine, 1,3-bis(aminomethyl)cyclohexane, 1,4-bis(aminomethyl)cyclohexane, isophoronediamine and 1-methylimidazole.

11. A curable composition according to claim 6, in which said adduct b) said polyepoxide has the formula II

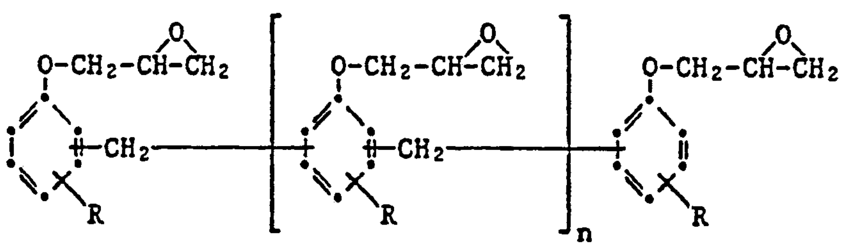

(II),

in which R is hydrogen or methyl, the methyl group is preferably in the ortho-position to the glycidyl ether group, and n is 0.2—3.4.

12. A curable composition according to claim 6, in which said adduct b) said polyepoxide has the formula III

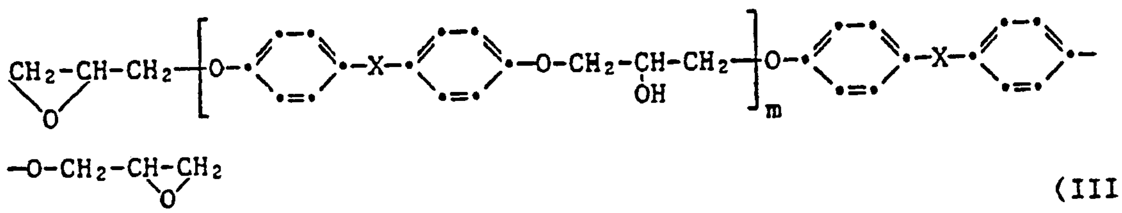

(III),

in which m is 0—50 and X is $—CH_2—$, $—C(CH_3)_2—$ or $—SO_2—$.

13. A curable composition according to claim 6, in which in said adduct b) said polyepoxide is a 1,4-buttanediol diglycidyl ether.

14. A curable composition according to claim 6, in which said adduct b) 2—20 moles of diamine are employed per mole of polyepoxide.

15. The cured product obtainable by curing a composition according to claim 6 at elevated temperatures.

16. The cured product obtainable by curing a composition according to claim 9 at elevated temperatures.

17. A curable composition containing a) a urethane resin and b) an adduct according to claim 1.

18. The cured product obtainable by curing a composition according to claim 17 at elevated temperatures.